# EUROPÄISCHE PATENTSCHRIFT

(11) **EP 1 337 298 B1**
(45) Veröffentlichungstag und Bekanntmachung des Hinweises auf die Patenterteilung: **22.09.2004**
(21) Anmeldenummer: 01969482.7
(22) Anmeldetag: 24.07.2001
(51) Int. Cl.: A61N 5/06, A61H 39/00

(54) **VORRICHTUNG ZUR AKUPUNKTUR MITTELS LASERSTRAHLUNG**
DEVICE FOR PERFORMING ACUPUNCTURE USING LASER RADIATION
DISPOSITIF D'ACUPONCTURE PAR RAYONNEMENT LASER

(30) Priorität: 20.11.2000 DE 20019703 U
(43) Veröffentlichungstag der Anmeldung: 27.08.2003
(73) Patentinhaber: Ronbar AG, 4051 Basel (CH)
(72) Erfinder: Schikora, Dr., Detlef, 37688 Wehrden (DE)
(74) Vertreter: Lorenz, Werner, Dr.-Ing.
(86) Internationale Anmeldenummer: PCT/EP2001/008504
(87) Internationale Veröffentlichungsnummer: WO 2002/040098

(56) Entgegenhaltungen:
- EP-A- 0 437 636
- EP-A- 0 495 757
- DE-A- 2 740 969
- DE-A- 3 729 288
- DE-A- 19 607 174
- US-A- 5 250 068
- US-A- 6 074 411

## Beschreibung

Die Erfindung betrifft eine Vorrichtung zur Akupunktur eines Patienten mittels Laserstrahlung, mit einer Einrichtung zur Erzeugung der Laserstrahlung und mit mehreren damit verbundenen Handstücken, welche dafür vorgesehen sind, mit dem Körper des Patienten in Berührung zu treten, um die Laserstrahlung aufzubringen.

Aus der DE 196 07 174 A1 ist eine Akupunkturnadel bekannt, die für subkutane Laserlichtapplikation verwendet wird. Die Nadel wird also in die Haut des Patienten eingestochen, um auf diese Weise tiefere Gewebeschichten zu erreichen. Nachteilig dabei ist, dass solche Nadeln bei Patienten mit Nadelphobie, bei Mensehen muslimischen Glaubens und insbesondere bei Kindern nicht eingesetzt werden können.

In der DE 37 29 288 A1 ist ein Lasergerät beschrieben, bei dem mehrere verschiedene Laserlichtquellen mittels Lichtleitfasern in einem Behandlungsstift zusammengeführt werden. Dieser Behandlungsstift ermöglicht nur eine aufeinanderfolgende Behandlung mehrerer Akupunkturpunkte und ist zur simultanen Reizung von Akupunktur-Punktkombinationen nicht geeignet.

Die Vorrichtung gemäß der EP 0 437 636 R1 beruht auf nicht kohärenter, thermischer Strahlung, die nach dem Prinzip der Reflexion in einem elektrischen Hohlspiegel gebündelt wird. Die höchste Energiedichte wird bei dieser Vorrichtung im Inneren im zweiten Brennpunkt des Hohlspiegels erreicht.

Die DE 27 40 969 A1 bezieht sich auf ein Faserbündel, das an den Enden in seine Bestandteile aufgespleißt wird. Die distalen Enden des Faserbündels sind mit Kunstoff-Flügeln versehen, die mittels Klebestreifen auf der Haut fixiert werden können. Allerdings ist die beschriebene Befestigung sehr unflexibel und es kann mit dieser Vorrichtung bei weitem keine ausreichende Akupunkturwirkung erzielt werden.

Weitere ähnliche Vorrichtungen zur Akupunktur mittels Laserstrahlung sind beispielsweise aus der EP 0 722 750 B1, der EP 0 416 150 B1, der DE 197 37 675 A1 oder der DE 89 11 606 U1 bekannt. Hierbei werden zum Erzeugen der Laserstrahlung häufig Halbleiter-Laserdioden mit verhältnismäßig niedriger Ausgangsleistung für einen Wellenlängenbereich von 630 bis 950 nm eingesetzt. Um einen kompakten Aufbau der Vorrichtung zu erreichen, sind die Einrichtungen zur Erzeugung der Laserstrahlung meist in dem Handstück untergebracht. Mit Hilfe einer Optik wird dann die Laserstrahlung an der Austrittsöffnung des Handstücks fokussiert.

Nachteilig hierbei ist jedoch, dass die von solchen Vorrichtungen emittierte Laserstrahlung eine solch geringe Intensität aufweist, dass die gesamte Energie bereits in den oberen Hautschichten des behandelten Patienten absorbiert und in den Kollagenfasern flächenhaft verteilt wird. Dadurch lässt sich auf die mit Akupunktur zu behandelnde Stelle kein ausreichender Reiz ausüben, der notwendig ist, um einen derartigen Behandlungserfolg zu erzielen, der mit demjenigen zu vergleichen ist, der bei der Behandlung mit üblichen Akupunkturnadeln eintritt.

Die beschriebenen Vorrichtungen sind somit allenfalls in der Dermatologie zur Heilung von Wunden und Narben anwendbar, können für die Reizung von Akupunkturpunkten jedoch nicht verwendet werden.

Bei der bereits seit geraumer Zeit auch im europäischen Raum bekannten Akupunktur mit üblichen Metallnadeln sind zum einen die bei der Behandlung eventuell auftretenden Sterilitätsprobleme nachteilig sowie die Tatsache, dass das Gewebe im Bereich der Akupunkturpunkte traumatisiert wird. Des weiteren können solche Metallnadeln bei Patienten mit. Nadelphobis und insbesondere bei Kindern nicht eingesetzt werden.

Es ist daher Aufgabe der vorliegenden Erfindung, eine Vorrichtung zur Akupunktur eines Patienten mittels Laserstrahlung zu schaffen, welche in der Lage ist, eine ausreichende Reizwirkung auch in denjenigen tiefer liegenden Hautschichten hervorzurufen, die erreicht werden müssen, damit eine Akupunkturbehandlung erfolgreich sein kann. Dabei soll die Vorrichtung möglichst einfach zu handhaben sein und eine bestmögliche Behandlung gewährleisten.

Erfindungsgemäß wird diese Aufgabe durch die in Anspruch 1 genannten Merkmale gelöst.

Durch die mit dem Körper des Patienten in Kontakt stehende Lichtleitfaser, die innerhalb des Handstücks angeordnet ist, kann die von der Einrichtung zur Erzeugung der Laserstrahlung ausgesandte elektromagnetische Energie in ihrer vollen Intensität in den Körper des Patienten eindringen und mit einer hohen, das Gewebe aber nicht verletzenden Energiedichte einen Reiz erzeugen.

Damit kann auf äußerst schonende Weise eine Akupunkturbehandlung durchgeführt werden, deren therapeutische Wirkung der Behandlung mit üblichen Metallnadeln zumindest ebenbürtig ist. Vorteilhafterweise lässt sich außerdem der Patientenkreis auch auf Personen ausweiten, die mit den üblichen Metallnadeln nicht behandelt werden konnten. Die erfindungsgemäße Vorrichtung kann somit in allen medizinischen Einrichtungen und bei Heilpraktikern problemlos eingesetzt werden, da zwar eine ausreichende, physiologisch jedoch unbedenkliche Reizwirkung eintritt.

Dadurch, dass der Querschnitt der wenigstens einen Lichtleitfaser im Bereich der Einrichtung zur Erzeugung der Laserstrahlung größer ist als in demjenigen Bereich, der bei der Akupunktur mit dem Körper des Patienten in Kontakt steht, erreicht man vorteilhafterweise bei gleicher Ausgangsleistung des Lasers eine Erhöhung der Energiedichte der Laserstrahlen, die die Reizwirkung auf der Körperoberfläche hervorruft. Dies eröffnet der erfindungsgemäßen Vorrichtung einen noch größeren Anwendungsbereich und es ist, je nach Auslegung des Querschnitts der Lichtleitfaser, möglich, in unterschiedlich tiefe Hautschichten einzudringen.

Insbesondere die Leistungseinsparung bei der Einrichtung zur Erzeugung der Laserstrahlung ist hier als vorteilhaft herauszustellen, da auf diese Weise verhältnismäßig kostengünstige Laser eingesetzt werden können und damit auch die gesamte Vorrichtung kostenmäßig in einem vertretbaren Rahmen bleibt.

Dadurch, dass mehrere Handstücke vorgesehen sind, welche jeweils eine Lichtleitfaser aufweisen, können, wie dies an sich in der klassischen Akupunktur gefordert wird, mehrere Stellen am Körper des Patienten gleichzeitig behandelt werden, so dass energetische Blockaden an verschiedenen Stellen im Meridiansystem aufgehoben werden können. Gegenüber den eingangs beschriebenen Laser-Akupunkturgeräten stellt dies einen erheblichen Vorteil dar, da eine gleichzeitige Behandlung mehrerer Akupunkturstellen nicht vorgesehen und auch nicht möglich war.

Ein Schutz für die Lichtleitfaser ergibt sich dadurch, dass das Handstück ein Gehäuse zur Aufnahme der Lichtleitfaser aufweist. In diesem Zusammenhang ist eine einfache und zugleich sehr sichere Anbringung des Handstücks an dem Körper des Patienten dadurch möglich, dass am äußeren Umfang des Gehäuses ein gegenüber dem Gehäuse verschiebliches Befestigungselement zur Anbringung des Handstücks an dem Körper des Patienten angeordnet ist.

Zur Verbindung der einzelnen in den einzelnen Handstücken untergebrachten Lichtleitfasern mit der Einrichtung zur Erzeugung der Laserstrahlung ergeben sich dann verschiedene Möglichkeiten.

Zum einen kann die Einrichtung zur Erzeugung der Laserstrahlung mehrere unabhängige Laserstrahlerzeugungsmittel aufweisen, wobei jede einzelne Lichtleitfaser mit jeweils einem Laserstrahlerzeugungsmittel verbunden ist. Diese in der Art eines Arrays dargestellte Ausführungsform ist nach dem Prinzip ausgeführt, dass jedes Laserstrahlerzeugungsmittel genau eine Lichtleitfaser versorgt, so dass nach Inbetriebnahme des Arrays die einzelnen Akupunkturpunkte stimuliert werden können und die Reizeinwirkung auf alle ausgewählten Akupunkturpunkte gleichzeitig erfolgt.

Alternativ hierzu kann die Einrichtung zur Erzeugung der Laserstrahlung wenigstens zwei Laserstrahlerzeugungsmittel aufweisen, welche über ein optisches Faserkopplungselement miteinander verschaltet sind, wobei jede einzelne Lichtleitfaser mit dem Faserkopplungselement verbunden ist.

Dabei werden die einzelnen Lichtleitfasern von dem optischen Faserkopplungselement versorgt, wodurch es möglich ist, jede einzelne Lichtleitfaser mit polychromatischer Laserstrahlung zu versorgen, d.h. mit Laserstrahlung von wenigstens zwei unterschiedlichen Wellenlängen, so dass die Absorption der Laserstrahlung in wenigstens zwei unterschiedlichen Gewebetiefen erfolgen kann. Selbstverständlich kann bei dieser Ausführungsform jede einzelne Lichtleitfaser auch mit monochromatischer Laserstrahlung versorgt werden.

Schließlich ergibt sich noch die Möglichkeit, dass die Einrichtung zur Erzeugung der Laserstrahlung ein Laserstrahlerzeugungsmittel aufweist, welches mit jeder der einzelnen Lichtleitfasern über ein Faserbündel verbunden ist. Bei dieser Ausführungsform lässt sich die einem derartigen Faserbündel innewohnende Flexibilität ausnutzen, um somit eine verhältnismäßig einfache Handhabung der einzelnen Handstücke zu erreichen.

Weitere vorteilhafte Ausgestaltungen und Weiterbildungen der Erfindung ergeben sich aus den restlichen Unteransprüchen sowie aus dem nachfolgend anhand der Zeichnung prinzipmäßig dargestellten Ausführungsbeispiel.

Es zeigt:
- Fig. 1: einen Schnitt durch ein Handstück der erfindungsgemäßen Vorrichtung zur Akupunktur;
- Fig. 2: eine erste Ausführungsform der erfindungsgemäßen Vorrichtung zur Akupunktur mit mehreren Handstücken;
- Fig. 3: eine zweite Ausführungsform der erfindungsgemäßen Vorrichtung zur Akupunktur mit mehreren Handstücken;
- Fig. 4: eine dritte Ausführungsform der erfindungsgemäßen Vorrichtung zur Akupunktur mit mehreren Handstücken; und
- Fig. 5: eine schematische Darstellung eines Steuergerätes für die erfindungsgemäße Vorrichtung.

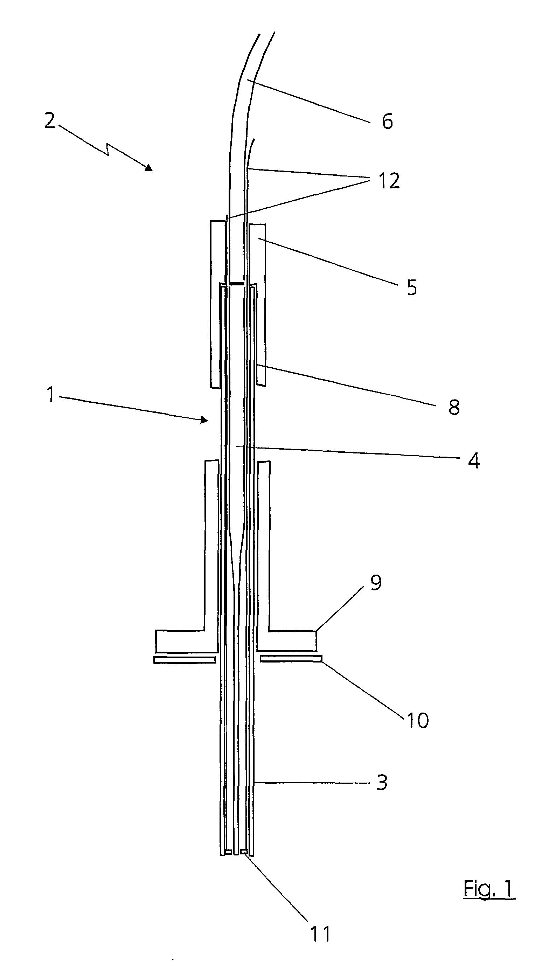

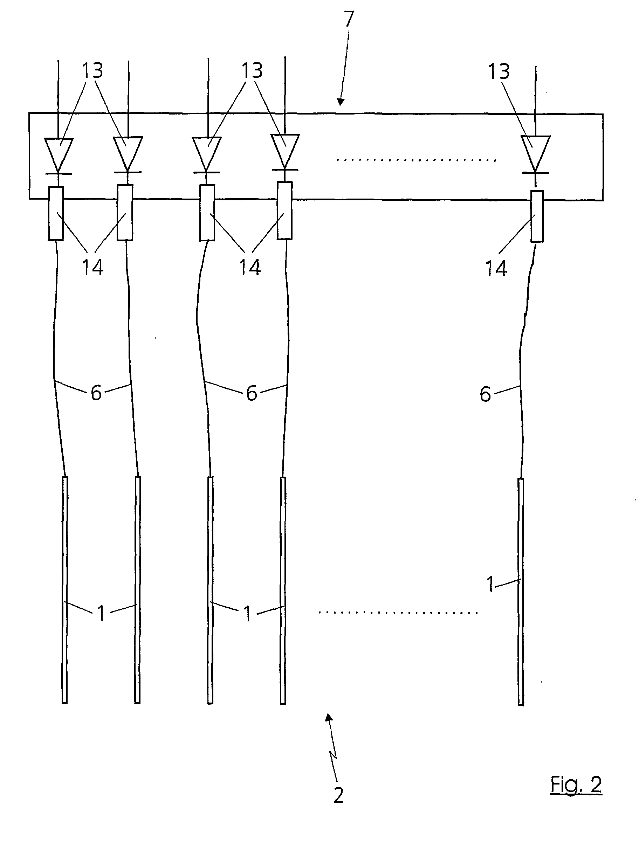

Fig. 1 zeigt ein Handstück 1 als Teil einer Vorrichtung 2 zur Akupunktur eines nicht dargestellten Patienten mittels Laserstrahlung. Der Aufbau der gesamten Vorrichtung 2 geht aus den Figuren 2, 3 und 4 hervor und wird unter Bezugnahme auf diese Figuren näher beschrieben.

Das Handstück 1 weist ein hülsenförmiges, zylindrisches Gehäuse 3 auf, in dessen Innerem eine Lichtleitfaser 4 untergebracht ist. Über ein optisches Kopplungselement 5 ist an der Lichtleitfaser 4 eine Verbindungs-Lichtleitfaser 6 angebracht, welche die Lichtleitfaser 4 mit einer erst in den Figuren 2, 3 und 4 dargestellten Einrichtung 7 zur Erzeugung von Laserstrahlung verbindet. Die von der Einrichtung 7 erzeugte Laserstrahlung kann somit über die Verbindungs-Lichtleitfaser 6 in die Lichtleitfaser 4 gelangen und, wenn das Handstück 1 auf den Körper des Patienten aufgesetzt wird, in dessen Hautschichten eindringen. Zur sicheren Verbindung der Lichtleitfaser 4 mit der Verbindungs-Lichtleitfaser 6 ist das optische Kopplungselement 5 im vorliegenden Fall mit einem Gewinde 8 versehen, um es an dem Gehäuse 3 anzubringen. Selbstverständlich sind hierzu auch andere Verbindungsmöglichkeiten denkbar.

Zur Anbringung bzw. Befestigung des Handstücks 1 an dem Körper des Patienten ist am äußeren Umfang des Gehäuses 3 ein Befestigungselement 9 angeordnet, welches mit dem Gehäuse 3 mechanisch verbindbar ist. Diese mechanische Verbindung wird im vorliegenden Fall dadurch geschaffen, daß das Befestigungselement 9 aus Gummi, beispielsweise aus Silikon, besteht und somit eine verhältnismäßig große Reibung zwischen dem aus einer Keramik, vorzugsweise einer Korundkeramik, bestehenden Gehäuse 3 herrscht. Selbstverständlich kann das Gehäuse 3 auch aus einem Leichtmetall, vorzugsweise aus Titan, bestehen, um bei sehr großer Festigkeit des Gehäuses 3 eine möglichst geringe Masse zu besitzen.

Alternativ zu der mechanischen Verbindung zwischen dem Befestigungselement 9 und dem Gehäuse 3 durch Reibung kann beispielsweise am Außenumfang des Befestigungs-elements 9 auch eine nicht dargestellte Klammer angebracht sein, die das Befestigungselement 9 fest an dem Gehäuse 3 anbringt. An der dem Körper des Patienten zugewandten Seite des Befestigungselements 9 befindet sich ein dermatologisch verträglicher, doppelt wirkender Klebering 10, der zur Anbringung am Körper des Patienten vorgesehen ist.

Soll also das Handstück 1 am Körper des Patienten befestigt werden, so wird das Befestigungselement 9 so lange entlang des Gehäuses 3 in Richtung des Körpers des Patienten verschoben, bis der Klebering 10 am Körper des Patienten zur Anlage kommt und dort klebt. Durch die mechanische Verbindung zwischen dem Befestigungselement 9 und dem Gehäuse 3 kann das Handstück 1 dann nicht mehr verrutschen und bleibt bis zum Lösen des Befestigungselementes 9 durch die behandelnde Person am Körper des Patienten.

Innerhalb des Gehäuses 3 befinden sich des weiteren zwei Elektroden 11, die zur Messung des Hautwiderstandes des Körpers des Patienten vorgesehen sind. Mit dieser an sich bekannten Messung des Hautwiderstandes ist es möglich, die für die Akupunktur notwendigen Punkte zu ermitteln bzw. auszuwählen. Die beiden Elektroden 11 sind jeweils mit Verbindungsleitungen 12 versehen, deren Funktion zu einem späteren Zeitpunkt näher erläutert wird.

Aus Fig. 1 geht des weiteren hervor, daß der Querschnitt der Lichtleitfaser 4 in dem Bereich des optischen Kopplungselementes 5 größer ist als in demjenigen Bereich, der bei der Akupunktur mit dem Körper des Patienten in Kontakt steht. Auf diese Weise wird die Energiedichte der über die Verbindungs-Lichtleitfaser 6 in die Lichtleitfaser 4 eingeleiteten Laserstrahlung umgekehrt proportional zu dem Verhältnis des Querschnitts an der Austrittsstelle zu dem Querschnitt an der Eintrittsstelle der Laserstrahlung erhöht. Durch diese höhere Energiedichte können auch tiefer liegende Akupunkturpunkte des Patienten mit der Laserstrahlung erreicht werden, was für verschiedene Arten der Akupunkturbehandlung zwingend erforderlich ist.

Die Verringerung der Querschnittsfläche der Lichtleitfaser 4 beträgt im vorliegenden Fall 1/3 bis 1/10, wobei an der Spitze der Lichtleitfaser 4 eine optisch aktive Fläche mit einer Ausdehnung von ca. 0,05 - 0,1 mm entsteht. Durch unterschiedliche Querschnittsänderungen der Lichtleitfaser 4 und der sich daraus ergebenden Veränderung der Energiedichte ergeben sich für die Laserstrahlung verschiedene Eindringtiefen, wie dies auf analoge Weise bei Metallnadeln für die klassische Akupunktur mit unterschiedlichen Längen erreicht wird.

In einer nicht dargestellten Ausführungsform kann auch auf die Verbindungs-Lichtleitfaser 6 verzichtet werden, was bedeutet, daß die Lichtleitfaser 4 dann direkt mit der Einrichtung 7 zur Erzeugung der Laserstrahlung verbunden ist. Die Verringerung des Querschnitts der Lichtleitfaser 4 kann sich prinzipiell über deren gesamte Länge verteilen.

Die Lichtleitfaser 4 weist einen koaxialen Aufbau mit einem nicht dargestellten Kern und einer ebenfalls nicht dargestellten Umhüllung auf. Hierbei kann der Kern der Lichtleitfaser 4 aus Quarzglas oder aus Kunststoff bestehen, abhängig von der Wellenlänge der zu übertragenden Laserstrahlung.

Dabei sind der Kern und die Umhüllung der Lichtleitfaser 4, wie bekannt, so aufgebaut, daß an der Umhüllung eine Totalreflexion der Laserstrahlung eintritt und die Lichtwelle den Kern der Lichtleitfaser 4 nicht verlassen kann. Dies muß selbstverständlich auch dann gelten, wenn sich der Querschnitt der Lichtleitfaser 4 verjüngt.

Die verwendeten Wellenlängenbereiche der Laserstrahlung können von nahem Ultraviolett bis zu nahem Infrarot variieren, so daß Wellenlängen von ca. 350 - 980 nm zur Anwendung kommen können. Hierbei hat sich herausgestellt, daß insbesondere Wellenlängen in einem Bereich von ca. 800 - 950 nm dafür geeignet sind, in tiefer liegende Hautregionen einzudringen. Auch durch die Variierung der verwendeten Wellenlänge der Laserstrahlung läßt sich somit eine Anpassung an die verschiedenen Körperbereiche und die jeweiligen therapeutischen Ziele der Akupunkturbehandlung erreichen, da unterschiedliche Wellenlängen auch unterschiedliche Eindringtiefen der Laserstrahlung zur Folge haben.

In Fig. 2 ist die gesamte Vorrichtung 2 dargestellt, die mit mehreren Handstücken 1 versehen ist. Im Durchschnitt wird es sich dabei um vier bis zehn Handstücke 1 handeln, je nach Art der Akupunkturbehandlung. Auf diese Weise können mehrere Punkte am Körper des Patienten gleichzeitig einer Akupunktur unterzogen werden, wie dies auch bei der klassischen Akupunktur mit Metallnadeln der Fall ist.

Hierbei weist die Einrichtung 7 eine der Anzahl der Handstücke 1 und somit der Lichtleitfasern 4 entsprechende Anzahl an Laserstrahlerzeugungsmitteln 13 auf, die unabhängig voneinander arbeiten und jeweils einzeln mit den Lichtleitfasern 4 verbunden sind. Als Laserstrahlerzeugungsmittel 13 sind prinzipiell Laserdioden, wie z.B. Gallium-Arsenit-Laserdioden oder Gallium-Nitrid-Laserdioden geeignet, die hierbei in Form eines Arrays angeordnet sind. Es sind dabei alternativ unterschiedliche oder auch identische Laserstrahlerzeugungsmittel 13 zur Erzeugung von Laserstrahlung mit entsprechenden Wellenlängen einsetzbar, so daß die einzelnen Handstücke 1 gegebenenfalls auch unterschiedliche Eindringtiefen in den Körper des Patienten hervorrufen können.

Jedem einzelnen Laserstrahlerzeugungsmittel 13 ist eine Kollimator- und Fokussieroptik 14 zugeordnet, von der die einzelnen Verbindungs-Lichtleitfasern 6 ausgehen, die dann schließlich, wie oben beschrieben, zu den einzelnen Lichtleitfasern 4 führen. Somit ist also die Einrichtung 7 zur Erzeugung der Laserstrahlung außerhalb des Handstücks 1 angeordnet, wodurch verhältnismäßig leistungsstarke Laserstrahlerzeugungsmittel 13 eingesetzt werden können, da diese nicht an die verhältnismäßig geringe Größe der einzelnen Handstücke 1 angepaßt werden müssen. Durch die oben beschriebene Verringerung des Querschnitts der einzelnen Lichtleitfasern 4 sind solche leistungsstarken Laserstrahlerzeugungsmittel 13 jedoch nicht unbedingt erforderlich.

Wie bereits oben erwähnt, können alternativ zu der Verbindung der Lichtleitfasern 4 über die Verbindungs-Lichtleitfaser 6 mit der jeweiligen Kollimator- und Fokussieroptik 14 die Lichtleitfasern 4 auch direkt mit der zugehörigen Kollimator- und Fokussieroptik 14 verbunden sein.

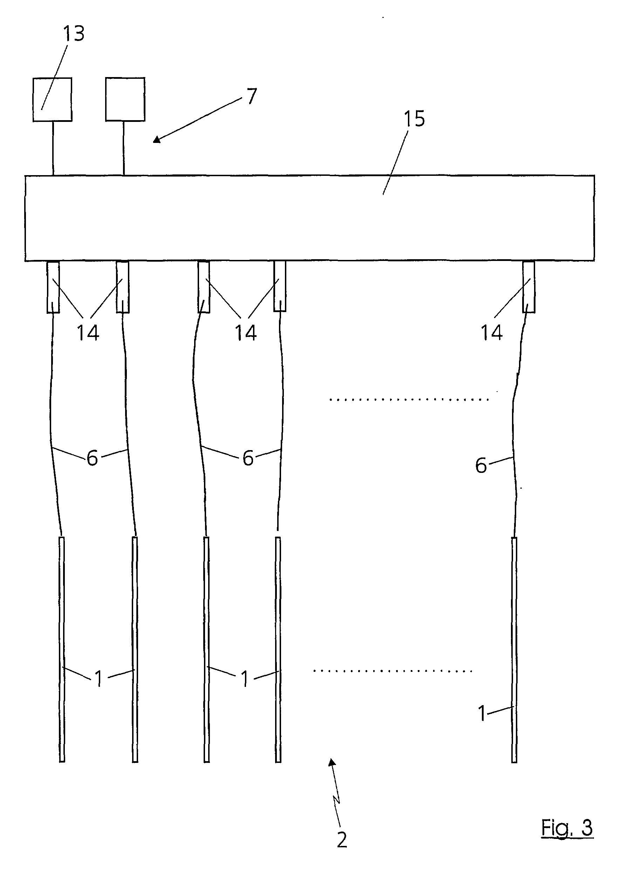

Bei der Ausführungsform der Vorrichtung 2 gemäß Fig. 3 weist die Einrichtung 7 zur Erzeugung der Laserstrahlung zwei voneinander unabhängige Laserstrahlerzeugungsmittel 13 auf, die beispielsweise als Gasentladungslaser oder als Festkörperlaser ausgebildet sein können. Selbstverständlich ist es auch möglich, die bereits oben beschriebenen Laserdioden einzusetzen und es kann auch eine andere Anzahl an Laserstrahlerzeugungsmitteln 13 verwendet werden. Die beiden Laserstrahlerzeugungsmittel 13 sind über ein optisches Faserkopplungselement 15, dessen Wirkungsweise an sich bekannt ist, miteinander verschaltet. Am Ausgang des Faserkopplungselementes 15 sind jeweils wiederum die oben beschriebenen Kollimator- und Fokussieroptiken 14 angeordnet. Von dort gehen, wie bei der Ausführungsform gemäß Fig. 2, jeweils die Verbindungs-Lichtleitfasern 6 zu den einzelnen Handstücken 1 mit den Lichtleitfasern 4 aus.

Bei dieser Vorrichtung 2 können über das optische Faserkopplungselement 15 die einzelnen Laserstrahlen der beiden Laserstrahlerzeugungsmittel 13 beliebig an die Lichtleitfasern 4 weitergegeben werden, wobei zuvor eine beliebige Vermischung stattfinden kann. Auf diese Weise läßt sich Laserstrahlung mit beliebiger Wellenlänge in den Körper des Patienten einbringen, und zwar sowohl als mono- als auch als polychromatische Laserstrahlung.

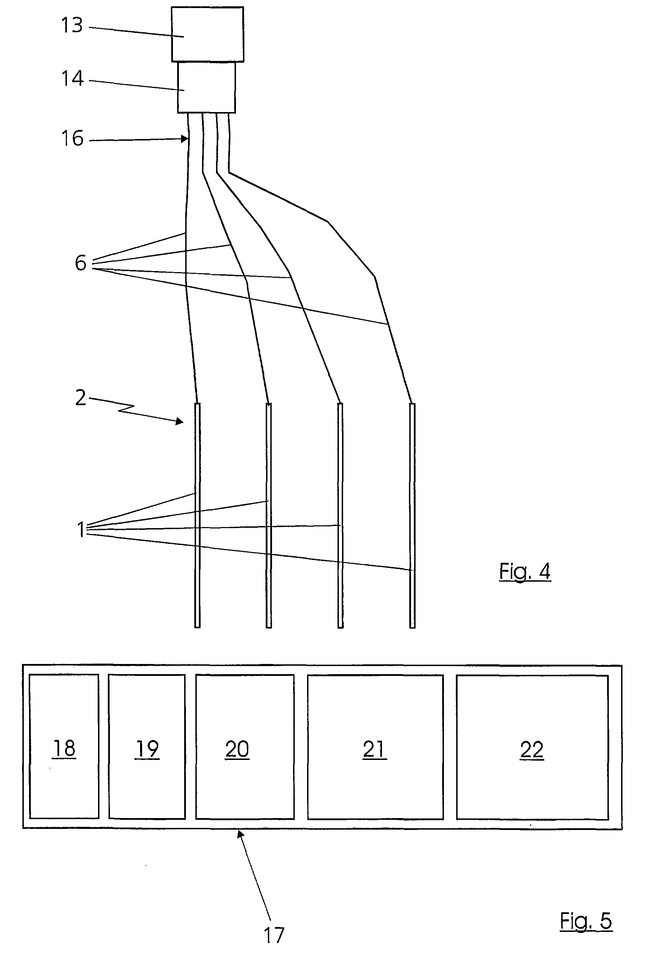

Bei der Vorrichtung 2 gemäß Fig. 4 weist die Einrichtung 7 zur Erzeugung der Laserstrahlung ein Laserstrahlerzeugungsmittel 13 auf, welches wiederum als Laserdiode, als Gasentladungslaser oder als Festkörperlaser ausgebildet sein kann und welches über eine einzelne Kollimator- und Fokussieroptik 14 und über ein Faserbündel 16 mit jeder der einzelnen Lichtleitfasern 4 der einzelnen Handstücke 1 verbunden ist. Alle Lichtleitfasern 4 werden also mit derselben Laserstrahlung beaufschlagt. Durch das Faserbündel 16 ergibt sich eine Flexibilität, die eine einfache Handhabung der einzelnen Handstücke 1 ermöglicht.

Fig. 5 zeigt ein Steuergerät 17, welches zum Steuern der Einrichtungen 7 zur Erzeugung der Laserstrahlung sowie für weitere im folgenden beschriebenen Funktionen vorgesehen ist. Hierzu weist das Steuergerät 17 ein Versorgungsmodul 18 auf, das die Netzspannung in eine zum Betreiben der Einrichtung 7 gegebenenfalls erforderliche Gleichspannung von 2 - 8 Volt umwandelt.

Des weiteren ist ein Modul 19 vorgesehen, welches zur Messung der Intensität der Laserstrahlung vor der Applikation dient und somit eine Anpassung an die unterschiedlichen Reizschwellen der einzelnen Patienten ermöglicht. Das Modul 19 kann somit auch als Photometer bezeichnet werden.

Ein weiteres Modul 20 dient zur zeitlichen Steuerung der Laserstrahlung und schaltet die jeweiligen Laserstrahlerzeugungsmittel 13 frei. Hierdurch läßt sich die Dauer der Akupunkturbehandlung einstellen und steuern.

Zur Modulation der Intensität der Laserstrahlung der Laserstrahlerzeugungsmittel 13 mit physiologisch relevanten Bahroder Nogierfrequenzen dient ein weiteres Modul 21 der Steuereinrichtung 17.

Schließlich ist noch ein Modul 22 zur Aufnahme der Einrichtung 7 zur Erzeugung der Laserstrahlung vorgesehen, welches bei den einzelnen Ausführungsformen gemäß der Figuren 2, 3 und 4 jeweils entweder das Laserdiodenarray aus den Laserstrahlerzeugungsmitteln 13, das optische Faserkopplungselement 15 oder gegebenenfalls auch das Faserbündel 16 aufnimmt.

Des weiteren können die von den Elektroden 11 ausgehenden Verbindungsleitungen 12 mit dem Steuergerät 17 verbunden sein, um ein Signal zu erzeugen, wenn ein Akupunkturpunkt aufgefunden wurde.

## Patentansprüche

1. Vorrichtung zur nicht-invasiven Akupunktur eines Patienten mittels Laserstrahlung, mit einer Einrichtung zur Erzeugung der Laserstrahlung und mit mehreren damit verbundenen Handstücken (1), welche dafür vorgesehen sind, mit dem Körper des Patienten in Berührung zu treten, um die Laserstrahlung aufzubringen, wobei ein Handstück (1) jeweils eine mit der Einrichtung (7) zur Erzeugung der Laserstrahlung verbundene Lichtleitfaser (4) aufweist, welche einen koaxialen Aufbau mit einer Umhüllung und einem Kern aufweist, dessen Querschnitt im Bereich der Einrichtung (7) zur Erzeugung der Laserstrahlung größer ist als in demjenigen Bereich, der bei der Akupunktur mit dem Körper des Patienten in Kontakt steht, so dass die Energiedichte an der Lichtaustrittsfläche am größten ist, wobei das Handstück (1) ein Gehäuse (3) aufweist, das die Lichtleitfaser (4) bis zur Lichtaustrittsfläche umschließt und am äußeren Ümfang ein gegenüber dem Gehäuse (3) verschiebbares Befestigungselement (9) enthält, das zur Anbringung des Handstücks (1) am Körper des Patienten dient.

2. Vorrichtung nach Anspruch 1,
**dadurch gekennzeichnet , dass**
die Lichtleitfaser (4) mit der Einrichtung (7) zur Erzeugung der Laserstrahlung über eine Kollimator- und Fokussieroptik (14) verbunden ist.

3. Vorrichtung nach Anspruch 1 oder 2,
**dadurch gekennzeichnet, dass**
die Einrichtung (7) zur Erzeugung der Laserstrahlung außerhalb des Handstücks (1) angeordnet ist.

4. Vorrichtung nach Anspruch 2 oder 3,
**dadurch gekennzeichnet, dass**
die Lichtleitfaser (4) direkt mit der Kollimator- und Fokussieroptik (14) verbunden ist.

5. Vorrichtung nach Anspruch 2 oder 3,
**dadurch gekennzeichnet, dass**
die Lichtleitfaser (4) über eine Verbindungs-Lichtleitfaser (6) mit der Kollimator- und Fokussieroptik (14) verbunden ist.

6. Vorrichtung nach Anspruch 5,
**dadurch gekennzeichnet, dass**
die Lichtleitfaser (4) ausschließlich in dem Handstück (1) verläuft und über ein optisches Kopplungselement (5) mit der Verbindungs-Lichtleitfaser (6) verbunden ist.

7. Vorrichtung nach einem der Ansprüche 1 bis 6,
**dadurch gekennzeichnet, dass**
die Einrichtung (7) zur Erzeugung der Laserstrahlung mehrere unabhängige Laserstrahlerzeugungsmittel (13) aufweist, wobei jede einzelne Lichtleitfaser (4) mit jeweils einem Laserstrahlerzeugungsmittel (13) verbunden ist.

8. Vorrichtung nach einem der Ansprüche 1 bis 6,
**dadurch gekennzeichnet, dass**
die Einrichtung (7) zur Erzeugung der Laserstrahlung wenigstens zwei Laserstrahlerzeugungsmittel (13) aufweist, welche über ein Faserkopplungselement (15) miteinander verschaltet sind, wobei jede einzelne Lichtleitfaser (4) mit dem Faserkopplungselement (15) verbunden ist.

9. Vorrichtung nach einem der Ansprüche 1 bis 8,
**dadurch gekennzeichnet , dass**
der Kern der Lichtleitfaser (4) aus Quarzglas besteht.

10. Vorrichtung nach einem der Ansprüche 1 bis 8,
**dadurch gekennzeichnet , dass**
der Kern der Lichtleitfaser (4) aus Kunststoff besteht.

11. Vorrichtung nach einem der Ansprüche 1 bis 10,
**dadurch gekennzeichnet, dass**
das Befestigungselement (9) mit dem Gehäuse (3) mechanisch verbindbar ist.

12. Vorrichtung nach Anspruch 11,
**dadurch gekennzeichnet, dass**
die mechanische Verbindung zwischen dem Befestigungselement (9) und dem Gehäuse (3) durch Reibung erfolgt.

13. Vorrichtung nach einem der Ansprüche 1 bis 12,
**dadurch gekennzeichnet, dass**
das Befestigungselement (9) aus Gummi besteht.

14. Vorrichtung nach einem der Ansprüche 1 bis 13,
**dadurch gekennzeichnet , dass**
an der dem Körper des Patienten zugewandten Seite des Befestigungselements (9) ein Klebering (10) zur Anbringung am Körper des Patienten angeordnet ist.

15. Vorrichtung nach einem der Ansprüche 1 bis 14,
**gekennzeichnet durch,**
ein Steuergerät (17) zum Steuern der Handstücke (1) und/oder der Einrichtungen (7) zur Erzeugung der Laserstrahlung, welches ein Versorgungsmodul (18), ein Modul (19) zur Messung der Intensität der Laserstrahlung, ein Modul (20) zur zeitlichen Steuerung der Laserstrahlung, ein Modul (21) zur Modulation der Intensität der Laserstrahlung und ein Modul (22) zur Aufnahme der Einrichtung (7) zur Erzeugung der Laserstrahlung aufweist.

16. Vorrichtung nach einem der Ansprüche 1 bis 15,
**dadurch gekennzeichnet, dass**
der Querschnitt des Kerns des Lichtwellenleiters (4) in demjenigen Bereich, der bei der Akupunktur mit dem Körper des Patienten in Kontakt steht, so verkleinert ist, dass die Lichtaustritts-Kontaktfläche immer der Ort der höchsten Energiedichte in dem Handstück (1) und in dem Lichtwellenleiter (4) ist.

17. Vorrichtung nach einem der Ansprüche 1 bis 16,
**dadurch gekennzeichnet, dass**
der Querschnitt des Kerns des Lichtwellenleiters (4) im Bereich der Einrichtung (7) zur Erzeugung der Laserstrahlung so vergrößert ist, dass die Lichteintrittsfläche immer der Ort der niedrigsten Energiedichte in dem Lichtwellenleiter (4) ist.

## Claims

1. Device for non-invasive acupuncture of a patient by means of laser radiation, comprising a means for generating a laser beam and comprising a plurality of handpieces (1) that are connected thereto and that are provided for coming into contact with the body of the patient in order to apply the laser radiation, wherein a handpiece (1) has in each case an optical fibre (4) connected to the means (7) for generating the laser radiation, which optical fibre has a coaxial structure with a sheath and a core whose cross section is greater in the region of the means (7) for generating the laser radiation than in that region that is in contact with the body of the patient during the acupuncture, with the result that the energy density is greatest at the light exit surface, wherein the handpiece (1) has a casing (3) that encloses the optical fibre (4) right up to the light exit surface and comprises, at the outer circumference, an attachment element (9) that is displaceable with respect to the casing (3) and that serves to apply the handpiece (1) to the body of the patient.

2. Device according to Claim 1, **characterized in that** the optical fibre (4) is connected to the means (7) for generating the laser radiation via a collimator and focusing optical system (14).

3. Device according to Claim 1 or 2, **characterized in that** the means (7) for generating the laser radiation is disposed outside the handpiece (1).

4. Device according to Claim 2 or 3, **characterized in that** the optical fibre (4) is connected directly to the collimator and focusing optical system (14).

5. Device according to Claim 2 or 3, **characterized in that** the optical fibre (4) is connected to the collimator and focusing optical system (14) via a connecting optical fibre (6).

6. Device according to Claim 5, **characterized in that** the optical fibre (4) extends exclusively in the handpiece (1) and is connected to the connecting optical fibre (6) via an optical coupling element (5).

7. Device according to any one of Claims 1 to 6, **characterized in that** the means (7) for generating the laser radiation comprise a plurality of independent laser-beam-generating means (13), wherein each individual optical fibre (4) is connected in each case to a laser-beam-generating means (13).

8. Device according to any one of Claims 1 to 6, **characterized in that** the means (7) for generating the laser radiation comprises at least two laser-beam-generating means (13) that are interconnected via a fibre coupling element (15), wherein each individual optical fibre (4) is connected to the fibre coupling element (15).

9. Device according to any one of Claims 1 to 8, **characterized in that** the core of the optical fibre (4) is composed of quartz glass.

10. Device according to any one of Claims 1 to 8, **characterized in that** the core of the optical fibre (4) is composed of plastic.

11. Device according to any one of Claims 1 to 10, **characterized in that** the attachment element (9) can be connected mechanically to the casing (3).

12. Device according to Claim 11, **characterized in that** the mechanical connection between the attachment element (9) and the casing (3) takes place by friction.

13. Device according to any one of Claims 1 to 12, **characterized in that** the attachment element (9) is composed of rubber.

14. Device according to any one of Claims 1 to 13, **characterized in that** an adhesive ring (10) for the application to the body of the patient is disposed at that side of the attachment element (9) facing the body of the patient.

15. Device according to any one of Claims 1 to 14, **characterized by** a controller (17) for controlling the handpieces (1) and/or the means (7) for generating the laser radiation, which controller comprises a supply module (18), a module (19) for measuring the intensity of the laser radiation, a module (20) for the temporal control of the laser radiation, a module (21) for modulating the intensity of the laser radiation and a module (22) for receiving the means (7) for generating the laser radiation.

16. Device according to any one of Claims 1 to 15, **characterized in that** the cross section of the core of the optical waveguide (4) is reduced **in that** region that is in contact with the body of the patient during the acupuncture in such a way that the light exit contact surface is always the point of highest energy density in the handpiece (1) and in the optical waveguide (4).

17. Device according to any one of Claims 1 to 16, **characterized in that** the cross section of the core of the optical waveguide (4) is increased in the region of the means (7) for generating the laser radiation in such a way that the light entrance surface is always the point of lowest energy density in the optical waveguide (4).

## Revendications

1. Dispositif d'acupuncture non invasive d'un patient à l'aide d'un rayon laser, avec un dispositif de production du rayon laser et plusieurs pièces à main (1) reliées à celui-ci, prévus pour entrer en contact avec le corps du patient afin d'appliquer le rayon laser, dans lequel une pièce à main (1) comprend respectivement une fibre optique (4), reliée au dispositif (7) de production du rayon laser, qui présente une structure coaxiale avec une enveloppe et un noyau, dont la section est supérieure, au niveau du dispositif (7) de production du rayon laser, à celle qui se trouve dans la zone qui entre en contact avec le corps du patient lors de l'acupuncture, de telle sorte que la densité d'énergie soit la plus importante au niveau de la surface de sortie de la lumière, la pièce à main (1) comprenant un boîtier (3) qui entoure les fibres optiques (4) jusqu'à la surface de sortie de la lumière et contient un élément de fixation (9), coulissant par rapport au boîtier (3), qui sert à l'application de la pièce à main (1) sur le corps du patient.

2. Dispositif selon la revendication 1,
**caractérisé en ce que**
la fibre optique (4) est reliée au dispositif (7) de production du rayon laser par l'intermédiaire d'une optique de collimation et de focalisait (14).

3. Dispositif selon la revendication 1 ou 2,
**caractérisé en ce que**
le dispositif (7) de production du rayon laser se trouve à l'extérieur de la pièce à main (1).

4. Dispositif selon la revendication 2 ou 3,
**caractérisé en ce que**
la fibre optique (4) est reliée directement à l'optique de collimation et de focalisation (14).

5. Dispositif selon la revendication 2 ou 3,
**caractérisé en ce que**
la fibre optique (4) est reliée à l'optique de collimation et de focalisation (14) par l'intermédiaire d'une fibre optique de liaison (6).

6. Dispositif selon la revendication 5,
**caractérisé en ce que**
la fibre optique (4) se trouve exclusivement dans la pièce à main (1) et est reliée à la fibre optique de liaison (6) par l'intermédiaire d'un élément de couplage optique (5).

7. Dispositif selon l'une des revendications 1 à 6,
**caractérisé en ce que**
le dispositif (7) de production du rayon laser comprend plusieurs moyens de production de rayon laser (13) indépendants, chaque fibre optique (4) étant reliée respectivement à un moyen de production de rayon laser (13).

8. Dispositif selon l'une des revendications 1 à 6,
**caractérisé en ce que**
le dispositif (7) de production du rayon laser comprend au moins deux moyens de production de rayon laser (13), reliés entre eux par l'intermédiaire d'un élément de couplage de fibres (15), chaque fibre optique (4) étant reliée à l'élément de couplage des fibres (15).

9. Dispositif selon l'une des revendications 1 à 8,
**caractérisé en ce que**
le noyau de la fibre optique (4) est constitué de verre au quartz.

10. Dispositif selon l'une des revendications 1 à 8,
**caractérisé en ce que**
le noyau de la fibre optique (4) est constitué de matière plastique.

11. Dispositif selon l'une des revendications 1 à 10,
**caractérisé en ce que**
l'élément de fixation (9) peut être relié mécaniquement au boîtier (3).

12. Dispositif selon la revendication 11,
**caractérisé en ce que**
la liaison mécanique entre l'élément de fixation (9) et le boîtier (3) est réalisée par frottement.

13. Dispositif selon l'une des revendications 1 à 12,
**caractérisé en ce que**
l'élément de fixation (9) est constitué de caoutchouc.

14. Dispositif selon l'une des revendications 1 à 13,
**caractérisé en ce que**,
du côté de l'élément de fixation (9) orienté vers le corps du patient, se trouve une bague adhésive (10) pouvant être appliquée sur le corps du patient.

15. Dispositif selon l'une des revendications 1 à 14,
**caractérisé par**
un appareil de commande (17) pour commander les pièces à main (1) et/ou les dispositifs (7) de production du rayon laser, qui comprend un module d'alimentation (18), un module (19) de mesure de l'intensité du rayon laser, un module (20) de commande dans le temps du rayon laser, un module (21) pour la modulation de l'intensité du rayon laser et un module (22) pour loger le dispositif (7) de production du rayon laser.

16. Dispositif selon l'une des revendications 1 à 15,
**caractérisé en ce que**
la section du noyau de la fibre optique (4) est réduite dans la zone en contact avec le corps du patient lors de l'acupuncture, de telle sorte que la surface de contact et de sortie de la lumière soit toujours l'endroit où la densité d'énergie est la plus importante dans la pièce à main (1) et dans la fibre optique (4).

17. Dispositif selon l'une des revendications 1 à 16,
**caractérisé en ce que**
la section du noyau de la fibre optique (4) est augmentée au niveau du dispositif (7) de production du rayon laser, de telle sorte que la surface d'entrée de la lumière soit toujours l'endroit où la densité d'énergie est la moins importante dans la fibre optique (4).
